Europäisches Patentamt
European Patent Office
Office européen des brevets

(19) (11) Veröffentlichungsnummer: **0 255 678 A1**

(12) # EUROPÄISCHE PATENTANMELDUNG

(21) Anmeldenummer: **87110852.8**

(51) Int. Cl.[4]: **C07C 127/22**, C07D 213/63

(22) Anmeldetag: **27.07.87**

(30) Priorität: **05.08.86 DE 3626503**

(43) Veröffentlichungstag der Anmeldung: **10.02.88 Patentblatt 88/06**

(84) Benannte Vertragsstaaten: **CH DE FR GB IT LI NL**

(71) Anmelder: **CELAMERCK GmbH & Co. KG**
**Binger Strasse 173**
**D-6507 Ingelheim am Rhein(DE)**

(72) Erfinder: **Becher, Hans-Manfred, Dr.**
**Pfarrer-Heberer-Strasse 5**
**D-6507 Ingelheim am Rhein(DE)**

(54) **Verfahren zur Herstellung von Benzoylharnstoffen.**

(57) Zur Herstellung überwiegend bekannter Benzoylharnstoffe aus entsprechenden Isocyanaten und Benzamiden verwendet man Toluol oder auch Chlorbenzol als Lösungsmittel bei Reaktionstemperaturen zwischen 100 und 120°C

EP 0 255 678 A1

## Verfahren zur Herstellung von Benzoylharnstoffen

Die Erfindung betrifft ein Verfahren zur Herstellung von Benzoylharnstoffen der Formel

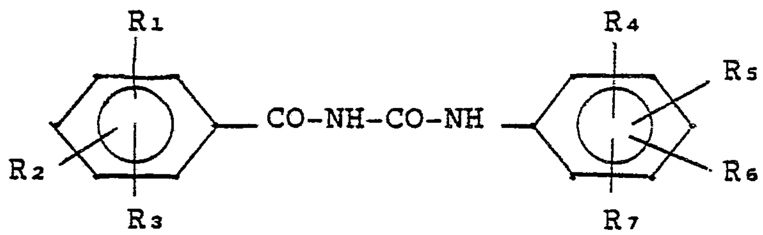

(I)

in der

$R_1$ Fluor, Chlor oder Methyl,

$R_2$ und $R_3$ (unabhängig voneinander) Wasserstoff, Fluor oder Chlor,

$R_4$ Fluor, Chlor, Brom, Methyl oder gegebenenfalls substituiertes Pyridyloxy,

$R_5$ Wasserstoff, Fluor oder Chlor

$R_6$ und $R_7$ (unabhängig voneinander) Fluor oder Chlor

bedeuten.

Die Substituenten in der Benzoylgruppe befinden sich bevorzugt in 2-und 6-Position. In der Anilinogruppe ist die eine ortho-Position vorzugsweise unsubstituiert.

Hervorzuheben sind diejenigen Verbindungen der Formel I, in denen die Benzoylgruppe disubstituiert ist, wobei sich die Substituenten, vorzugsweise Fluor, in 2-und 6-Position befinden.

Die bevorzugte Substitution der Anilinogruppe entspricht der Formel

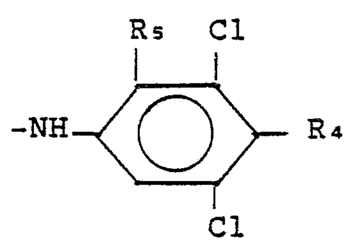

(II)

worin $R_5$ und $R_4$ Fluor oder Chlor; oder $R_5$ Wasserstoff und $R_4$ Fluor, Chlor, Brom oder auch Methyl bedeuten. Gleichzeitig ist die Benzoylgruppe in erster Linie 2,6-Difluorbenzoyl.

Besonders hervorgehoben sei die Verbindung der Formel

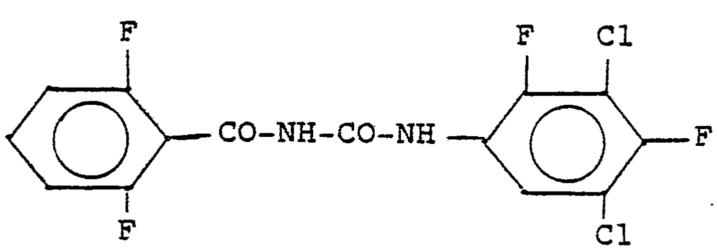

(III)

Die Verbindungen der Formel I sind größtenteils bekannt.

Es ist auch bekannt, solche Verbindungen durch Addition entsprechend substituierter Benzoylamide an entsprechend substituierte Phenylisocyanate herzustellen. Die Umsetzung wird in der Regel in inerten organischen Lösungsmitteln in der Wärme vorgenommen.

Es hat sich jedoch gezeigt, daß diese Umsetzung, wie sie z.B. in der EP-A 1 0 052 833 beschrieben ist, nicht völlig befriedigt. Bei höheren Temperaturen treten z.T.

Zersetzungen ein, bei niedriger Temperatur ist die Reaktionszeit nach technischen Maßstäben zu lang. Wie die genauere Untersuchung zeigt, enthalten die nach dem erwähnten Verfahren hergestellten Produkte erhebliche Anteile an Verunreinigungen. So ist z.B. das gemäß Beispiel 1 der oben zitierten EP-A 1 mit 94 % Ausbeute erhaltene Produkt nur knapp 90-prozentig, so daß die Ausbeute an dem gewünschten Benzoylharnstoff weniger als 85 % beträgt.

Wie nun gefunden wurde, lassen sich die Benzoylharnstoffe der Formel I in höherer Ausbeute und größerer Reinheit herstellen, wenn man die Isocyanate der Formel

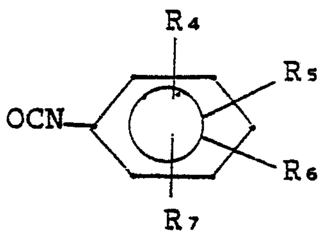

(IV),

in der $R_4$, $R_5$, $R_6$ und $R_7$ die obige Bedeutung haben, in Toluol oder auch in Chlorbenzol bei Temperaturen zwischen 100 und 120°C vorzugsweise 105 und 115°C mit den Benzamiden der Formel

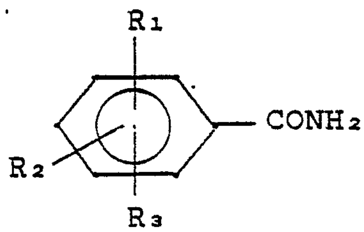

(V),

in der $R_1$, $R_2$ und $R_3$ die obige Bedeutung haben, umsetzt, wobei zwischen 3,5 und 12, vorzugweise zwischen 4 und 10 ml Lösungsmittel pro Gramm des Benzamids verwendet werden.

Optimal ist ein solches Mengenverhältnis von Lösungsmittel und Benzoylamid, daß wenigstens für eine kurze Zeit eine weitgehend klare Lösung entsteht. Andererseits ist eine möglichst hohe Konzentration an umzusetzendem Produkt in der Lösung anzustreben, um eine günstige Reaktionsgeschwindigkeit zu erreichen.

Im Fall der Addition von 2,6-Difluorbenzamid an halogen-, gegebenenfalls auch methylsubstituierte Phenylisocyanate liegt das Optimum bei 4 - 12 ml, vorzugsweise 6 - 10 ml Toluol pro Gramm des Amids.

Lösungsmittelmengen im oberen Teil des angegebenen Bereichs wirken sich auf die Produktqualität günstig aus.

Als besonders zweckmäßig hat es sich erwiesen, das als Ausgangsstoff benötigte Isocyanat der Formel IV in situ herzustellen, wobei - ausgehend vom entsprechend substituierten Benzoylfluorid - über das entsprechende Azid und Curtiusabbau das Isocyanat erhalten werden kann; Reaktionsschema:

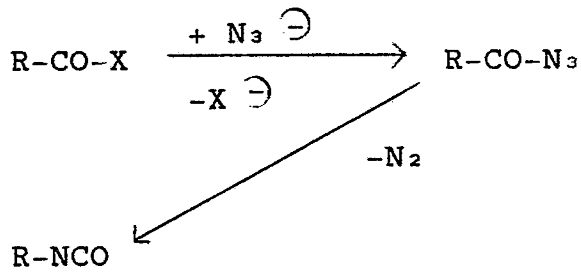


wobei R für die Gruppe

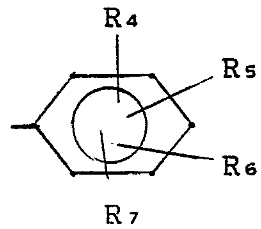

und X für eine gegen die Azid-Ion austauschbare Gruppe steht, z.B. Fluor, Chlor, Brom, Jod oder $-O-R_8$, mit $R_8$ gleich $C_1$-$C_4$-Niederalkyl, Phenyl, Benzyl, $C_1$-$C_4$-Niederalkoxycarbonyl, Phenoxycarbonyl, $C_1$-$C_4$-Niederalkylcarbonyl oder Benzoyl. Bevorzugt sind die Halogene Fluor und Chlor. Als Azide eignen sich z.B. Natriumazid, Kaliumazid und Calciumazid.

Das Benzoylamid und das Isocyanat werden zweckmäßig im Molverhältnis 1 : 1 eingesetzt. Da beim Curtiusabbau keine quantitative Ausbeute erzielt wird, hat es sich in der Praxis bewährt, einen Unterschuß an Benzoylamid, bezogen auf eingesetztes Benzoylfluorid, anzuwenden. Zweckmäßig ist ein Verhältnis Benzoylamid zu Benzoylfluorid von etwa 0,9 : 1 (0,85 bis 0,95 : 1).

<u>Beispiel 1</u>

N-(3,5-Dichlor-2,4-difluor-phenyl)-N'-(2,6-difluorbenzoyl)-harnstoff

Zu der Lösung von 16,8 g (75 mMol) 3,5-Dichlor-2,4-difluor-phenylisocyanat [1]) in 55 ml absolutem Toluol werden 11,8 g (75 mMol) 2,6-Difluorbenzamid [2]) gegeben. Die so erhaltene Mischung wird unter Rühren auf 105°C aufgeheizt und dann 7 bis 8 Stunden lang bei 105°C gerührt. Nach Erreichen der Reaktionstemperatur bildet sich kurzzeitig eine klare Lösung, aus der das Produkt schon während der Umsetzung auskristallisiert.

Danach wird das Reaktionsgemisch auf 10-15°C abgekühlt, das Ungelöste abgesaugt und bei 50°C im Umlufttrockenschrank getrocknet.
Rohausbeute: 27,2 g (95,1 % t.q.)
Fp.: 217 - 223°C
Zusammensetzung [3]) :
95,1 Gew.-% N-(3,5-Dichlor-2,4-difluor-phenyl)-N'-(2,6-difluorbenzoyl)-harnstoff
2,2 Gew.-% 2,6-Difluorbenzamid
2,5 Gew.-% Bis-(3,5-Dichlor-2,4-difluor-phenyl)harnstoff

[1]) Das Isocanat soll möglichst rein sein. Wenn es aus 3,5-Dichlor-2,4-difluor-anilin durch Phosgenieren hergestellt wurde, ist Destillation angebracht.

[2]) Das 2,6-Difluorbenzamid darf höchstens 0,1 % $H_2O$ enthalten.

[3]) Durch HPLC bestimmt.

Beispiel 2

N-(2,4-Difluor-3,5-dichlorphenyl)-N'-(2,6-difluorbenzoyl)harnstoff

a) 2,4-Difluor-3,5-dichlorbenzoylfluorid erhält man in über 70 % Ausbeute durch Umsetzung von 2,3,4,5-Tetrachlorbenzoylchlorid mit Kaliumfluorid in Sulfolan. Die Temperatur beträgt 150°C, die Reaktionszeit 8 Stunden.

b) Umsetzung von 2,4-Difluor-3,5-dichlorbenzoylfluorid mit Natriumazid

2,4-Difluor-3,5-dichlorbenzoylfluorid (11,45 g) werden zu einer auf etwa 110°C erwärmten Suspension von Natriumazid (3,25 g) in Toluol (40 ml) getropft, wobei der Reaktionsverlauf durch das freiwerdende Stickstoffgas kontrolliert wird, Nach Beendigung der Zugabe des Säurefluorids wird noch eine Stunde bei 110°C gerührt, wobei sich Natriumfluoridkristalle niederschlagen.

Die Lösung des erhaltenen 2,4-Difluor-3,5-dichlorphenylisocyanats kann unmittelbar für die weitere Umsetzung entsprechend Beispiel 1 verwendet werden. Ausbeute an >98%iger Titelverbindung: 86 % über beide Stufen.

## Ansprüche

1. Verfahren zur Herstellung von Benzoylharnstoffen der Formel

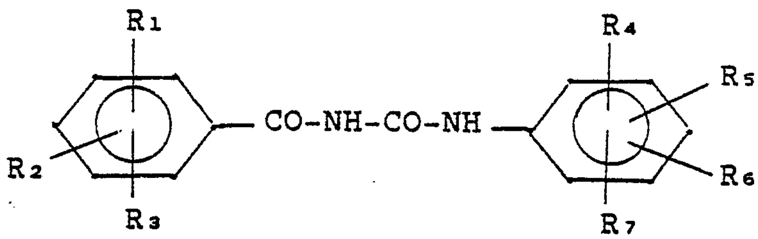

(I)

in der

$R_1$ Fluor, Chlor oder Methyl,

$R_2$ und $R_3$ (unabhängig voneinander) Wasserstoff, Fluor oder Chlor,

$R_4$ Fluor, Chlor, Brom, Methyl oder gegebenenfalls substituiertes Pyridyloxy,

$R_5$ Wasserstoff, Fluor oder Chlor

$R_6$ und $R_7$ (unabhängig voneinander) Fluor oder Chlor

bedeuten, dadurch gekennzeichnet, daß man ein Phenylisocyanat der Formel

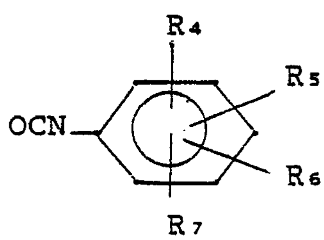

(IV),

mit dem Benzamid der Formel

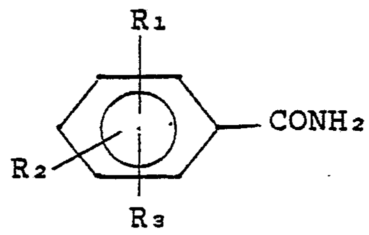

(V),

in Toluol oder Chlorbenzol bei Temperaturen zwischen 100 und 120, vorzugsweise zwischen 105 und 115°C umsetzt.

2. Verfahren nach Anspruch 1, dadurch gekennzeichnet, daß man das Phenylisocyanat der Formel IV in situ durch Curtius-Abbau des entsprechenden Azids herstellt und anschließend die Umsetzung mit dem Benzamid der Formel V durchführt.

3. Verfahren nach Anspruch 1 oder 2, dadurch gekennzeichnet, daß man das Phenylisocyanat der Formel IV, ausgehend von dem entsprechenden Benozylfluorid über das Benzoylazid durch Curtius-Abbau in situ hersetellt und das Benzamid der Formel V in geringem Unterschuß (Molverhältnis etwa 0,9 : 1), bezogen auf eingesetztes Benzoylfluorid, einsetzt.

Europäisches Patentamt

# EUROPÄISCHER RECHERCHENBERICHT

Nummer der Anmeldung

EP 87110852.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| P,X | WO - A1 - 86/05 487 (CELAMERCK) * Ansprüche 1,2; Seite 6, Zeilen 16-20 * -- | 1-3 | C 07 C 127/22<br>C 07 D 213/63 |
| X | EP - A1 - 0 176 868 (CELAMERCK) * Ansprüche 1,4,8; Beispiel 5a * -- | 1-3 | |
| X | DE - A1 - 3 123 720 (CELAMERCK) * Zusammenfassung; Seite 5 - Seite 6, Zeile 4 * -- | 1 | |
| D,X | EP - A1 - 0 052 833 (CELAMERCK) * Zusammenfassung; Seite 2 - Seite 3, Zeile 9; Beispiel 1 * -- | 1 | RECHERCHIERTE SACHGEBIETE (Int. Cl.4) |
| A | DE - A1 - 3 003 112 (CIBA-GEIGY) * Ansprüche 1,7; Seite 6, Zeilen 1-9 * -- | 1 | C 07 C 127/00<br>C 07 D 213/00 |
| P,A | EP - A2 - 0 197 280 (BAYER) * Ansprüche 1,6; Spalte 8, Zeilen 27-35; Spalte 8, Zeile 51 - Spalte 9, Zeile 2 * -- | 1 | |
| A | EP - A2 - 0 077 759 (CIBA-GEIGY) * Zusammenfassung; Spalte 2, Zeile 12 - Seite 3, Zeile 20 * ---- | 1 | |

Der vorliegende Recherchenbericht wurde für alle Patentansprüche erstellt.

| Recherchenort | Abschlußdatum der Recherche | Prüfer |
|---|---|---|
| VIENNA | 09-10-1987 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument